(19) Europäisches Patentamt
European Patent Office
Office européen des brevets

(11) Publication number: 0 021 772
B1

(12) **EUROPEAN PATENT SPECIFICATION**

(45) Date of publication of patent specification: **02.01.85**

(51) Int. Cl.[4]: **C 07 D 227/04**, C 07 D 227/087

(21) Application number: **80302033.8**

(22) Date of filing: **17.06.80**

(54) **Method of preparing 2-oxo- and 2-unsubstituted-hexahydroazepine derivatives.**

(30) Priority: **03.07.79 GB 7923123**

(43) Date of publication of application:
**07.01.81 Bulletin 81/01**

(45) Publication of the grant of the patent:
**02.01.85 Bulletin 85/01**

(84) Designated Contracting States:
**AT BE CH DE FR IT LI LU NL SE**

(56) References cited:
**DE-A-2 408 532**
**GB-A-1 186 481**
**GB-A-1 186 660**
**GB-A-1 198 973**
**GB-A-1 285 025**
**GB-A-1 346 625**
**GB-A-1 478 817**
**US-A-2 524 643**

**Ang.Chem. 1960, 72, 91-108**

**The file contains technical information submitted after the application was filed and not included in this specification**

(73) Proprietor: **JOHN WYETH & BROTHER LIMITED**
**Huntercombe Lane South Taplow**
**Maidenhead Berkshire, SL6 0PH (GB)**

(72) Inventor: **Bushell, Brian John**
**26 Durham Gardens Waterlooville**
**Portsmouth Hampshire (GB)**
Inventor: **Cavalla, John Frederick**
**105 The Grove**
**Isleworth Middlesex (GB)**
Inventor: **Shepherd, Robin Gerald**
**79 Huntercombe Lane North**
**Burnham Buckinghamshire (GB)**
Inventor: **White, Alan Chapman**
**5 Sherbourne Drive**
**Windsor Berkshire (GB)**

(74) Representative: **Brown, Keith John Symons et al**
**c/o John Wyeth & Brother Limited Patent and Trademark Department Huntercombe Lane South**
**Taplow Maidenhead Berkshire SL6 0PH. (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 021 772 B1

Courier Press, Leamington Spa, England.

**Description**

This invention relates to hexahydroazepine derivatives. More particularly the invention relates to a novel process for preparing 2-oxo-hexahydroazepine derivatives and their optional conversion to 2-unsubstituted-hexahydroazepine derivatives.

The 2-oxo-hexahydroazepine derivatives prepared by the novel process of the present invention have the general formula (Ia)

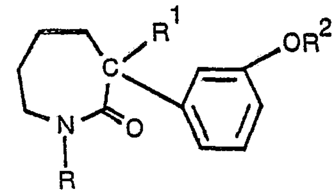

Ia

where R is hydrogen, lower alkyl, aryl(lower)alkyl, loweralkenyl-methyl or cycloalkylmethyl, $R^1$ is lower alkyl and $R^2$ is hydrogen, lower alkyl or aryl(lower)alkyl wherein "lower" defines a radical containing 1—6 C-atoms and "aryl" means a phenylgroup optionally substituted by alkyl or alkoxy.

These derivatives may be converted to the pharmacologically active 2-unsubstituted-hexahydroazepine derivatives of general formula (II)

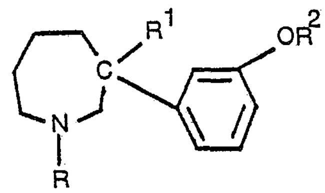

II

where R, $R^1$ and $R^2$ have the meanings given above. The compounds of general formula (Ia) may be reduced (e.g. with a hydride transfer agent such as lithium aluminium hydride) to the compounds of general formula (II). Compounds of general formula (II) in which $R^2$ is lower alkyl or aryl(lower)alkyl may be ether cleaved (e.g. with hydrogen bromide or boron tribromide) to compounds in which $R^2$ is hydrogen. The use of the compounds of formula (II) and their preparation from compounds of formula (Ia) is further exemplified in, for example, GB—A—1,285,025 and EP—A—0 003 253. The present invention provides a novel process for preparing the compounds of general formulae (Ia) and (II) via a benzyne derivative. The addition of benzynes to certain carbanions is known from, for example, Ang. Chem. 1960, *72*, 91—108.

According to the present invention there is provided a method of preparing a hexahydroazepine of general formula (I)

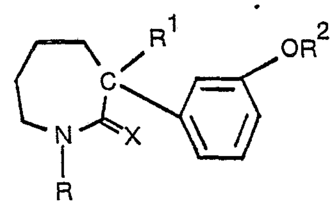

I

wherein

X is =0 or 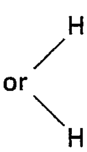

R is hydrogen, lower alkyl, aryl(lower)alkyl, lower alkenylmethyl or cycloalkylmethyl, $R^1$ is lower alkyl and $R^2$ is hydrogen, lower alkyl or aryl(lower)alkyl wherein "lower" defines a radical containing 1—6 C-atoms and "aryl" means a phenyl group optionally substituted by alkyl or alkoxy which comprises (a) reacting a lactam derivative of general formula (III)

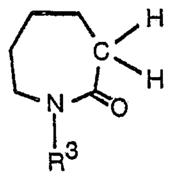

III

where $R^3$ is $C_{1-6}$-alkyl, phenyl-$C_{1-6}$-alkyl optionally substituted in the phenyl-moiety by alkyl or alkoxy, trialkyl-, triphenyl- or triphenylalkylsilyl, $C_{2-6}$-alkenyl-methyl or cycloalkylmethyl with a base which is a sodium, potassium or lithium secondary amide derived from a dialkylamine, dicyclohexylamine, t-butylcyclohexylamine, N-isopropyl-N-cyclohexylamine, N,N-(1'-ethylcyclohexyl)-1,1,3,3-tetramethylbutylamine, piperidine or 2,2,6,6-tetramethyl-piperidine to give an anion of the lactam derivative and reacting the anion with a benzyne of general formula (IV)

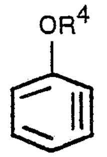

IV

(where $R^4$ is $C_{1-6}$-alkyl, phenyl-$C_{1-6}$-alkyl optionally substituted in the phenyl-moiety by the alkyl or alkoxy or trialkyl-, triphenyl- or triphenylalkylsilyl) prepared *in situ* by reacting a substituted halobenzene of general formula (V)

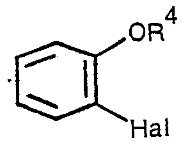

V

(where $R^4$ is as defined above and Hal is chlorine, bromine or iodine) with a base as defined above,
(b) reacting the resulting anion of step (a) with a $C_{1-6}$-allkylating agent, and
(c) protonating the resulting anion of the compound of the general formula (VI)

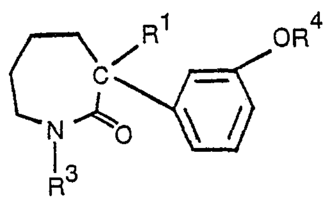

VI

where $R^1$, $R^3$ and $R^4$ have the meanings given above *in situ* to give a compound of general formula (I) wherein X is =0, and
(d) if desired, reducing the compound of general formula (I) wherein X is =0 to a compound of general formula (I) wherein

X is 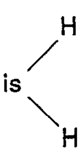 and,

if desired ether cleaving the compound of formula (I) wherein

X is 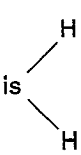 and

$R^2$ is $C_{1-6}$-alkyl or phenyl $C_{1-6}$-alkyl optionally substituted in the phenyl-moiety by alkyl or alkoxy to give a compound in which $R^2$ is hydrogen.

It will be realised that compounds of formula (I) in which X is =0 are the 2-oxo derivatives of formula (Ia) and compounds of formula (I) in which

X is 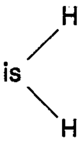

are the 2-unsubstituted compounds of formula (II).

$C_{1-6}$ alkyl radicals preferably contain 1 to 4 carbon atoms. For example R or $R^2$ may be methyl, ethyl, propyl, or butyl. Similarly $R^1$ may be, for example, methyl, ethyl, propyl or butyl. When R, $R^2$, $R^3$ or $R^4$ is phenyl $C_{1-6}$-alkyl, the radical is preferably phenethyl or benzyl; the phenyl group may be substituted by alkyl or alkoxy. When R or $R^3$ is $C_{2-6}$-alkenyl-methyl the radical is preferably allyl. When R or $R^3$ is cycloalkylmethyl the radical is preferably cyclopropylmethyl or cyclobutylmethyl.

The metal amide is preferably formed *in situ* by reacting a metal compound $MR^5$ (where M is sodium, potassium or lithium and $R^5$ is alkyl, aryl or aralkyl) with an appropriate secondary amine. The secondary amine is a dialkylamine (e.g. diethylamine, di-isopropylamine, di-tertiarybutylamine or N-*t*-amyl-N-*t*-butylamine), dicyclohexylamine, *t*-butyl-cyclohexylamine, N-isopropyl-N-cyclohexylamine, N-(1′-ethylcyclohexyl)-1,1,3,3-tetramethylbutylamine piperidine or 2,2,6,6-tetramethylpiperidine. Preferably the anion of the lactam is formed by reacting the lactam with lithium 2,2,6,6-tetramethylpiperidide (which may be prepared *in situ* from 2,2,6,6-tetramethylpiperidine and, for example, butyl lithium).

The benzyne of general formula (IV) is formed *in situ* by reaction of a substituted halobenzene of general formula (V)

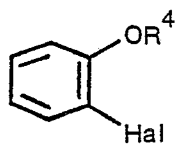

V

where $R^4$ is as defined above and Hal is chlorine, bromine or iodine (preferably chlorine) with a base as defined above. Preferably the base is lithium 2,2,6,6-tetramethylpiperidide (which as mentioned above may be formed *in situ*).

In an especially preferred process the formation of the anion of the lactam (III) and the formation of the benzyne is carried out in the same reaction vessel. For example, a base such as lithium 2,2,6,6-tetramethylpiperidine (formed *in situ*) may be reacted with a lactam of formula (III) to give the anion of the lactam and further lithium 2,2,6,6-tetramethylpiperidide may be generated *in situ* (by for example adding an alkyl lithium such as butyl lithium) and the substituted halobenzene (V) then added. The addition of the required quantity of substituted halobenzene (V), together with the further generation of the lithium 2,2,6,6-tetramethylpiperidide or other base, may be carried out in two or more separate additions.

The anion of step (a) may be reacted with a $C_{1-6}$ alkylating agent, for example a $C_{1-6}$-alkyl halide (preferably a $C_{1-6}$ alkyl iodide), to give the anion of the compound of formula (VI). This anion may then be protonated, e.g. by reaction with water, without isolation. For example, it may be protonated by reaction with water or dilute aqueous mineral acid (e.g. dilute hydrochloric acid) e.g. an excess of water may be added to the reaction vessel or the reaction mixture may be added to the water.

When $R^3$ and/or $R^4$ is trialkyl-, triphenyl- or triphenylalkylsilyl the hydrolysis of the product gives a compound of formula (I) in which R and/or $R^2$ is hydrogen. The hydrolysis may occur during protonation of the anion of the compound of formula (VI) by reaction with water.

The lactams of general formula (III) are known compounds or can be prepared by known methods, e.g. by N-"alkylation" of corresponding lactams in which $R^3$ is hydrogen.

The following Example illustrates the invention:

## Example

3-Ethylhexahydro-3-(3-methoxyphenyl)-1-methyl-2H-azepin-2-one

A solution of butyl lithium (0.05 mole) in hexane (31.25 ml) was treated with 2,2,6,6-tetramethylpiperidine (8.5 ml), followed by dry tetrahydrofuran ("THF"; 100 ml) under an inert atmosphere at 0°C. The mixture was treated with a solution of N-methylcaprolactam (6.25 ml, 50 mM) in THF (20 ml) and stirred for 30 minutes. A solution of butyl lithium (0.05 mole) in hexane (31.25 ml), was added and the mixture stirred for 15 minutes. A solution of o-chloroanisole (6.2 ml) in THF (20 ml) was added slowly, the mixture stirred for 1.5 hours and treated with ethyl iodide (5.15 ml). After one hour, the reaction was quenched with water (100 ml) and the solvents removed under reduced pressure. The residue was partitioned between 2N HCl (200 ml) and toluene (250 ml). The organic layer was washed with brine, dried and the solvents removed under reduced pressure to give 3-ethylhexahydro-3-(3-methoxyphenyl)-1-methyl-2*H*-azepin-2-one (5.5 g) b.p. 140°/0.1 mm. Recrystallisation from diisopropyl ether gave pure title compound (5.0 g) m.p. 68—69°C identical with authentic material.

**Claims**

1. A method of preparing a hexahydroazepine of general formula (I)

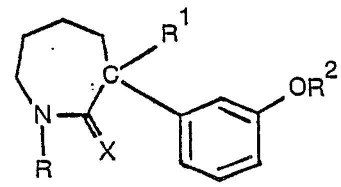

I

wherein

X is =O or 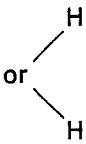

R is hydrogen, lower alkyl, aryl(lower)alkyl, lower alkenyl-methyl or cycloalkylmethyl, $R^1$ is lower alkyl and $R^2$ is hydrogen, lower alkyl or aryl(lower)alkyl wherein "lower" defines a radical containing 1—6 C-atoms and "aryl" means a phenylgroup optionally substituted by alkyl or alkoxy which comprises

(a) reacting a lactam derivative of general formula (III)

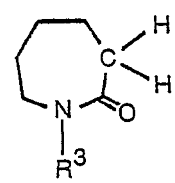

III

where $R^3$ is $C_{1-6}$-alkyl, phenyl-$C_{1-6}$-alkyl, optionally substituted in the phenyl moiety by alkyl or alkoxy trialkyl-, triphenyl- or triphenylalkylsilyl, $C_{2-6}$-alkenyl-methyl or cycloalkylmethyl

with a base which is a sodium, potassium or lithium secondary amide derived from a dialkylamine, dicyclohexylamine, t-butylcyclohexylamine, N-isopropyl-N-cyclohexylamine, N,N-(1′-ethylcyclohexyl)-1,1,3,3-tetramethylbutylamine, piperidine or 2,2,6,6-tetramethylpiperidine to give an anion of the lactam derivative and

reacting the anion with a benzyne of general formula (IV)

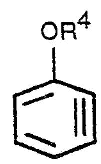

IV

(where $R^4$ is $C_{1-6}$-alkyl, phenyl-$C_{1-6}$-alkyl optionally substituted in the phenyl-moiety by alkyl or alkoxy or trialkyl-, triphenyl- or triphenylalkylsilyl) prepared *in situ* by reacting a substituted halobenzene of general formula (V)

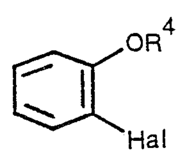

V

(where $R^4$ is as defined above and Hal is chlorine, bromine or iodine) with a base as defined above,
(b) reacting the resulting anion of step (a) with a $C_{1-6}$-alkylating agent, and
(c) protonating the resulting anion of the compound of general formula (VI

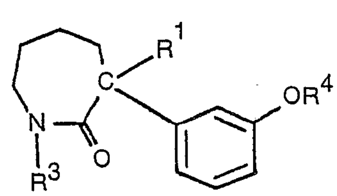

VI

where $R^1$, $R^3$ and $R^4$ have the meanings given above *in situ* to give a compound of general formula (I) wherein X is =0, and
(d) if desired, reducing the compound of general formula (I) wherein X is =0 to a compound of general formula (I) wherein

X is 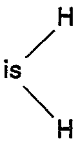 and,

if desired ether cleaving the compound of formula (I) wherein

X is 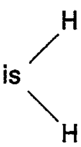 and

$R^2$ is $C_{1-6}$-alkyl or phenyl $C_{1-6}$-alkyl optionally substituted in the phenyl-moiety by alkyl or alkoxy to give a compound in which $R^2$ is hydrogen.

2. A method as claimed in Claim 1 wherein the base is lithium 2,2,6,6-tetramethylpiperidine.
3. A method as claimed in Claim 1 or 2 wherein $R^1$ is ethyl.
4. A method as claimed in any one of Claims 1 to 3 wherein R is methyl.

**Patentansprüche**

1. Verfahren zum Herstellen eines Hexahydroazepins der allgemeinen Formel (I)

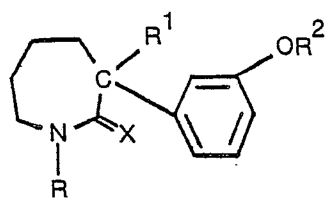

I

worin

X =0 oder 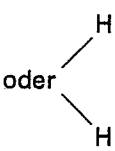 ist,

R Wasserstoff, nied.Alkyl, Aryl(nied.)alkyl, nied.Alkenylmethyl oder Cycloalkylmethyl bedeutet, $R^1$ nied.Alkyl darstellt und $R^2$ Wasserstoff, nied.Alkyl oder Aryl-(nied.)alkyl ist, wobei "nied." einen Rest mit 1 bis 6 C-Atomen definiert und "Aryl" eine Phenylgruppe bedeutet, die gegebenenfalls durch Alkyl oder Alkoxy substituiert ist, welches umfaßt:
(a) das Umsetzen eines Lactamderivats der allgemeinen Formel (III)

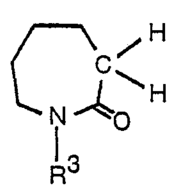

III

worin $R^3$ $C_{1-6}$-Alkyl, Phenyl-$C_{1-6}$-alkyl, in der Phenylgruppe gegebenenfalls substituiert durch Alkyl oder Alkoxy, Trialkyl-, Triphenyl- oder Triphenylalkylsilyl, $C_{2-6}$-Alkenylmethyl oder Cycloalkylmethyl ist, mit einer Base, die ein Natrium-, Kalium- oder Lithium-sek.amid ist, das von einem Dialkylamin, Dicyclohexylamin, tert.Butylcyclohexylamin, N-Isopropyl-N-cyclohexylamin, N,N-(1′-Äthylcyclohexyl)-1,1,3,3-tetramethylbutylamin, Piperidin oder 2,2,6,6-Tetramethylpiperidin abgeleitet ist, wobei ein Anion des Lactamderivats erhalten wird, und das Umsetzen des Anions mit einem Benzyn der allgemeinen Formel (IV)

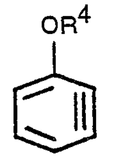

IV

(worin $R^4$ $C_{1-6}$-Alkyl, Phenyl-$C_{1-6}$-alkyl, in der Phenylgruppe gegebenenfalls substituiert durch Alkyl oder Alkoxy, oder Trialkyl-, Triphenyl- oder Triphenylalkylsilyl bedeutet), hergestellt in situ durch Umsetzen eines substituierten Halogenbenzols der allgemeinen Formel (V)

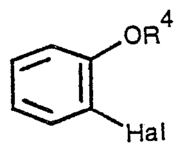

V

(worin $R^4$ wie oben definiert ist und Hal Chlor, Brom oder Jod bedeutet) mit einer Base, wie oben definiert,
(b) das Umsetzen des resultierenden Anions von Schritt (a) mit einem $C_{1-6}$-Alkylierungsmittel und
(c) das Protonieren des resultierenden Anions der Verbindung der allgemeinen Formel (VI)

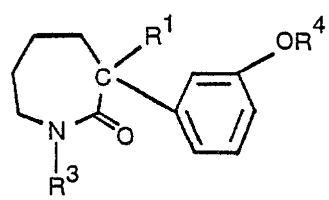

VI

worin $R^1$, $R^3$ und $R^4$ die oben angegebenen Bedeutungen haben, in situ, wobei eine Verbindung der allgemeinen Formel (I) erhalten wird, worin X die Bedeutung =O hat, und
(d) wenn gewünscht, das Reduzieren der Verbindung der allgemeinen Formel (I), worin X =O ist, zu einer Verbindung der allgemeinen Formel (I), worin

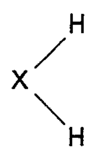

ist, und

wenn gewünscht, Ätherspaltung der Verbindung der Formel (I), worin

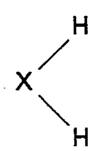

ist,

und $R^2$ $C_{1-6}$-Alkyl oder Phenyl-$C_{1-6}$-alkyl, in der Phenylgruppe gegebenenfalls durch Alkyl oder Alkoxy substituiert, ist, wobei eine Verbindung erhalten wird, worin $R^2$ Wasserstoff ist.

2. Verfahren wie in Anspruch 1 beansprucht, worin die Base Lithium-2,2,6,6-tetramethylpiperidid ist.

3. Verfahren wie in Anspruch 1 oder 2 beansprucht, worin $R^1$ Äthyl ist.

4. Verfahren wie in einem der Ansprüche 1 bis 3 beansprucht, worin R Methyl ist.

**Revendications**

1. Procédé de préparation d'une hexahydroazépine de formule générale:

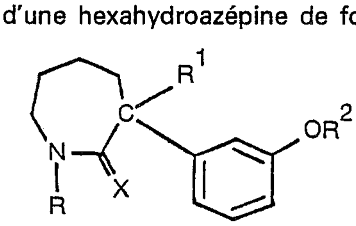

I

où X représente =0 ou 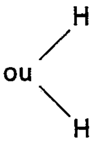

R représente un atome d'hydrogène ou radical alcoyle inférieur, arylalcoyle inférieur, alcényl(inférieur)-méthyle ou cycloalcoyl-méthyle, $R^1$ représente un radical alcoyle inférieur et $R^2$ représente un atome d'hydrogène ou radical alcoyle inférieur ou arylalcoyle inférieur, où le terme "inférieur" qualifie un radical comptant 1 à 6 atomes de carbone et "aryle" signifie un radical phényle éventuellement substitué par alcoyle ou alcoxy, qui comprend

(a) la réaction d'un dérivé de lactame de formule générale (III):

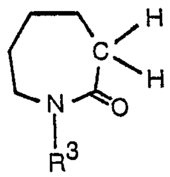

III

où $R^3$ représente un radical $C_{1-6}$-alcoyle, phényl-$C_{1-6}$-alcoyle éventuellement substitué sur la partie phényle par alcoyle ou alcoxy, trialcoyl-, triphényl- ou triphénylalcoylsilyle, $C_{2-6}$-alcényl-méthyle ou cycloalcoylméthyle, avec une base qui est un amidure secondaire de sodium, de potassium ou de lithium issu d'une dialcoylamine, de la dicyclohexylamine, de la t-butyl-cyclohexylamine, de la N-isopropyl-N-cyclohexylamine, de la N,N-(1'-éthylcyclohexyl)-1,1,3,3-tétraméthylbutylamine, de la pipéridine ou de la 2,2,6,6-tétraméthylpipéridine pour donner un anion du dérivé de lactame et

la réaction de l'anion avec un benzyne de formule générale (IV):

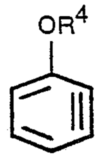

IV

(où $R^4$ représente un radical $C_{1-6}$-alcoyle, phényl-$C_{1-6}$-alcoyle éventuellement substitué sur la partie phényle par alcoyle ou alcoxy, ou trialcoyl-, triphényl- ou triphénylalcoylsilyle), préparé *in situ* par réaction d'un halogénobenzène substitué de formule générale (V):

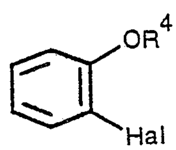

V

(où $R^4$ est tel que défini ci-dessus et Hal représente un atome de chlore, de brome ou d'iode), avec une base telle que définie ci-dessus,

(b) la réaction de l'anion résultant du stade (a) avec un agent de $C_{1-6}$-alcoylation, et

(c) la protonation de l'anion résultant du composé de formule générale (VI):

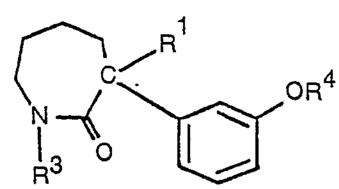

VI

où $R^1$, $R^3$ et $R^4$ ont les significations attribuées ci-dessus, *in situ* pour donner un composé de formule générale (I) où X représente =0, et

(d) si la chose est souhaitée, la réduction du composé de formule générale (I) où X représente =0 en un composé de formule générale (I) où

X représente 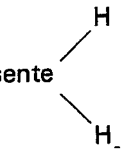

et, si la chose est souhaitée, la scission de la liaison éther du composé de formule (I) où

X représente 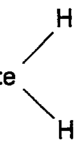

et $R^2$ représente un radical $C_{1-6}$-alcoyle ou phényl-$C_{1-6}$-alcoyle éventuellement substitué dans la partie phényle par alcoyle ou alcoxy, pour donner un composé où $R^2$ représente un atome d'hydrogène.

2. Procédé suivant la revendication 1, dans lequel le base est le 2,2,6,6-tétraméthylpipéridure de lithium.

3. Procédé suivant la revendication 1 ou 2, dans lequel $R^1$ représente un radical éthyle.

4. Procédé suivant l'une quelconque des revendications 1 à 3, dans lequel R représente un radical méthyle.